# EUROPEAN PATENT APPLICATION

(11) **EP 1 604 969 A1**
(43) Date of publication of application: **14.12.2005**
(21) Application number: 05253474.0
(22) Date of filing: 06.06.2005
(51) Int. Cl.: C07C 51/41, C07C 53/126

(54) **High shear process for making metallic esters**

(30) Priority: 07.06.2004 US 521618 P
(71) Applicant: Dover Chemical Corporation, Dover, Ohio 44622 (US)
(72) Inventor: Larke, Carroll W., Zoar OH 44697 (US)
(74) Representative: Scott, Susan Margaret

(57) **Abstract**

A process to make a metallic ester which comprises:
(a) mixing at least one metallic salt with at least one partially heated acidic organic moiety form a heated slurry at a first temperature;
(b) reacting said slurry under shear conditions to form a micro-emulsion for no longer than about two minutes at a second higher temperature to form a pumpable metallic ester; and
(c) collecting said metallic ester.

## Description

### Technical Field

This invention relates generally to a short residence time process for the manufacture of metallic esters in general and specifically, metallic soaps from the reaction of a metallic salt with an acid, preferably a fatty acid under conditions of high shear. In one embodiment of the invention, clear zinc stearate is prepared by reacting molten stearic acid with solid zinc oxide to form a slurry which forms a microemulsion under conditions of high shear to form zinc stearate with a reactor residence time of under one minute.

### Background of the Invention

Metallic soaps have found wide application in industry, for example, as waterproofing agents, thickening and suspending agents, and as lubricants; they are also employed in cosmetics, lacquers, plastics, in powder metallurgy, as mold release agents, flattening agents, fillers, anti-foaming agents and driers in paints, and in tablet manufacture. They are also used as heat and light stabilizers for plastics, especially polyvinyl chloride. The most common metallic soaps are those prepared from calcium, zinc, magnesium, barium and aluminum.

The heavy metal metallic soaps have conventionally been prepared from metal oxides or metal salts and aliphatic carboxylic acids, particularly the higher fatty acids containing from about 12 to 22 carbon atoms, which acids are known and sold to the industry as commercial fatty acids. The commercial fatty acids as commonly used are usually mixtures of higher fatty acids in which the name attached to them may be only the dominant acid of the mixture. In some grades of commercial stearic acid, however, the dominant fatty acid is not stearic acid but another fatty acid, for example, palmitic acid.

Metallic soaps of the higher fatty acids, the most common of which are the metallic stearates derived from commercial grades of stearic acid, are prepared by two principal methods: (i) the double decomposition process; and (ii) the reaction of metals, metal oxides, or metal hydroxides with molten fatty acids.

The double decomposition process is the oldest process and is still commonly used. A hot aqueous solution of the sodium salt of the fatty acid is first prepared by the addition of aqueous caustic soda to a mixture of the fatty acid in hot water. The sodium salt is then reacted with a hot aqueous solution of an appropriate metal salt. The insoluble precipitate of the fatty acid metallic soap is filtered, washed free of the soluble sodium salt, dried, and ground to a fine powder. By proper control of the reaction conditions, including temperature, rate of addition of reactants, and degree of dilution, a product with a fine particle size and of high purity can be obtained. The production cost however, is high, especially because of the filtration, washing, and drying operations required. The process also has the drawback that frequently it creates a water pollution problem. Zinc stearate is manufactured commercially employing this process by the action of sodium stearate on a solution of zinc sulfate.

There are several variations of the reaction of metals, metal oxides or metal hydroxides with molten fatty acids to form metallic soaps. In the fusion process, only certain metallic soaps are capable of being formed. The metallic soap is formed by reacting the molten fatty acid with the appropriate metal oxide or hydroxide at a temperature above the melting point of the metallic soap to be formed, and generally at a temperature considerably above this, because, during the reaction, water is formed and this must be driven off. Generally, the reaction requires about five hours for completion. This process can only be used for making metallic soaps which are pourable in their molten state. It, therefore, cannot be used for such metallic soaps as calcium or barium stearate, but it is suitable for zinc and lead stearates. The final product is in the form of flakes or lumps and a considerable energy must be expended in grinding it to the required very fine particle size. This process does have the advantage of not requiring the use of caustic soda, and no filtering or drying is required. It also does not lead to any water pollution or the consumption of any water.

The modified fusion process is much like the fusion process, except that a small amount of water is added to the mixture of molten fatty acid and metal oxide or hydroxide. The water acts as a catalyst and allows the reaction to be carried out at a somewhat lower temperature, and more quickly. The final product produced is very much like, if not identical to, that resulting from the fusion process.

Another variation employs fusion in an aqueous slurry. In this process, the molten fatty acid is first emulsified in water using an appropriate emulsifying agent. To this aqueous emulsion, is added an aqueous slurry of the metal oxide or hydroxide. The metallic oxide or hydroxide reacts with the fatty acid to form the metallic soap. This is then removed by filtration, during which it is washed, and then it is dried and ground. The product is considerably easier to grind because it is produced in the form of coarse particles. These particles, however, are much coarser than those produced in the double decomposition process.

Finally, occasionally, it is possible and of commercial value to react certain metals directly with molten fatty acid. For example, iron stearate may be prepared by this method; however, hydrogen rather than water is actually a by-product of reaction and, because the reaction generally has to be carried out at an unusually high temperature, the color of the resulting metallic soap is poor.

A specific example of the fusion process is illustrated in United States Patent No. 4,060,535 published November 29, 1977 which teaches that the fusion process has several disadvantages that limit its use in commercial applications, particularly in that it requires the use of expensive high temperature equipment and complicated handling procedures. Long reaction periods at elevated temperatures are represented to be necessary to allow the reaction to go to completion and that a discolored molten product is produced that on cooling forms into large lumps. A grinding operation is then required to convert the lumps into fine powder that is commercially acceptable. The '535 patent then teaches that to overcome the above problems, a reaction mixture of organic acid, metal component and a small amount of water, coupled with vigorous agitation in an apparatus having attrition and shearing action at a temperature that is below the melting point of the organic acid component (69.6°C) and below the melting point of the metal salt that is being produced (130°C) until substantially all of the organic acid component has reacted, is required. The process is represented to be capable of being carried out in any suitable apparatus in which particles of the reactants are continually subdivided under conditions of shear and attrition at relatively low temperatures and in which external cooling can be provided whenever necessary to maintain the temperature of the reaction mixture in the desired range. Vessels such as a Waring blender, Henschel fluid mixer and Littleford mixer were indicated to result in the successful formation of product. The patent further teaches that it is essential that between 0.1 % and 8.0% water be added based on the total weight of metal component and organic acid component since water acts as the initiator for the salt-forming reaction. When less water is added, the reaction takes place too slowly to be commercially acceptable and often does not go to completion.

United States Patent No. 4,307;027 issued December 22, 1981 teaches a synthesis using a continuous process employing the feeding of a fatty acid such as stearic acid and a base such as ZnO into a plug flow reaction using a residence time of between 2 to 60 minutes at a temperature between 75°F (24°C) to 280°F (138°C). The stirred tank reaction is a standard vertical, dished bottom tank which can operate between 60°F (16°C) to 300°F (149°C) and should be equipped with baffles, agitator, top feed nozzles and a bottom discharge. The plug flow reactor was represented to be almost any elongated system capable of heat transfer and/or shear energy generation and continuously moving material of a consistency that may vary from a viscous liquid to a paste to a solid. A Baker Perkins M-P mixer was indicted to be acceptable.

United States Patent No. 4,473,504 issued September 25, 1984 teaches the fusion reaction is still commercially carried out at a high temperature above the melting point of the metallic soap, approximately 130°C. Additionally, it is known that undesirable side reactions will occur interfering with the formation of high purity products, so that this method has not been used as widely as would have been expected. The '504 patent uses a water insoluble metal carbonate starting material.

United States Patent No. 5,164,523 issued November 17, 1992 teaches a reaction in which a melted fatty acid is added to a metal oxide. A catalyst is employed which induces fusion between the metal oxide and the fatty acid at a lower temperature than would otherwise be possible. The catalyst is represented to provide an induction temperature of about 125-150°C. The fatty acid, metal oxide and catalyst components preferably include no water. A spiral tube reaction is employed in this patent. By conducting the fusion reaction completely in the liquid phase, without the use of water, simplified equipment and reactor design are possible. No milling is necessary and no isolating, washing or drying steps are needed. The product is indicated to be clear, uncolored and stable. Exposure to high temperatures, i.e., those above the melting point of the soap, is brief (generally less than 5 minutes), and thermal degradation of the soap is minimized.

However, the prior art fails to teach any high shear reaction where a micro-emulsion of the reactants is produced in a high energy, dispersing environment, at a temperature which is just below the fusion initiation temperature. The invention recognizes the need for maintaining the temperature below the decomposition temperature of metallic soap (e.g., zinc stearate), which is achieved by a residence time in the reactor which is very short (preferably less than 1 minute) which is coupled with a high shear synthetic environment.

### Summary of the Invention

Accordingly it is a principal object of the invention to provide an alternative to existing fusion processes and which in one specific embodiment, incorporates the elements of molten stearic acid, ZnO slurry at a temperature just below the fusion temperature, a high shear reactor, and short residence time.

More generically, the invention involves the reaction of a polyvalent metal in its salt form with a molten organic acid or anhydride to form a slurry with subsequent reaction in a high shearing environment to form the metallic ester.

It is a further object of the invention to provide a process for producing a metallic soap having a viscosity such that it is a pourable liquid when molten, which is the reaction of an acidic organic moiety, preferably a carboxylic acid or anhydride wherein the organic component is a linear or branched, saturated or unsaturated, unsubstituted or substituted aliphatic hydrocarbon radical of 2 - 32 carbon atoms with a metallic oxide, carbonate or hydroxide.

It is a still further object of the invention to provide a process for producing a metallic fatty acid soap in which the fatty acid and metal component contain substantially no water.

It is a yet a further object of the invention to provide a process which does not employ a catalyst.

It is yet another object of the invention to provide a process for producing a metallic fatty acid soap for which the residence time in the reactor is less than two minutes, preferably less than one minute.

These and other objects of the present invention will become more readily apparent from a reading of the following detailed description taken in conjunction with the accompanying drawings and with further reference to the appended claims.

### Brief Description of the Drawings

The invention may take physical form in certain parts and arrangements of parts, a preferred embodiment of which will be described in detail in the specification and illustrated in the accompanying drawings which form a part hereof, and wherein:
*FIG. 1* is a process flow chart for the manufacture of zinc stearate in accordance with the invention;

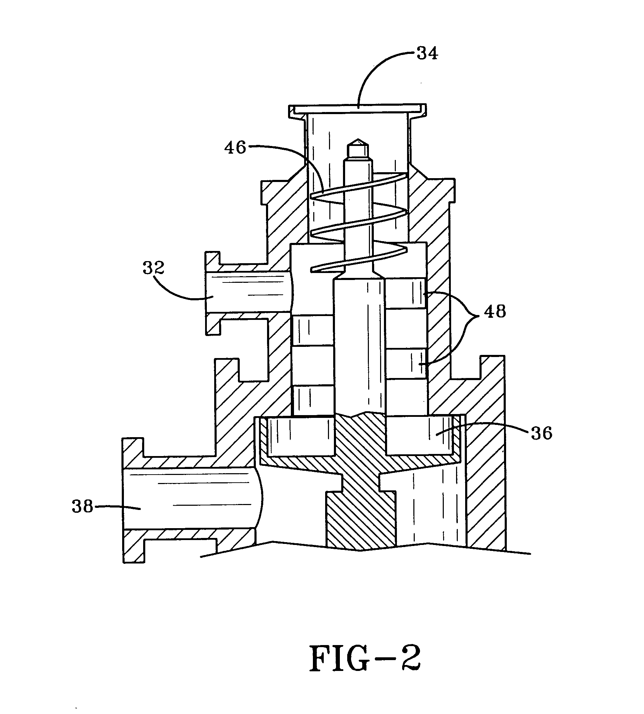

*FIG. 2* is an exploded view of the slurry mixer; and
*FIG. 3* is an exploded view of the high shear reactor.

### Detailed Description of the Invention

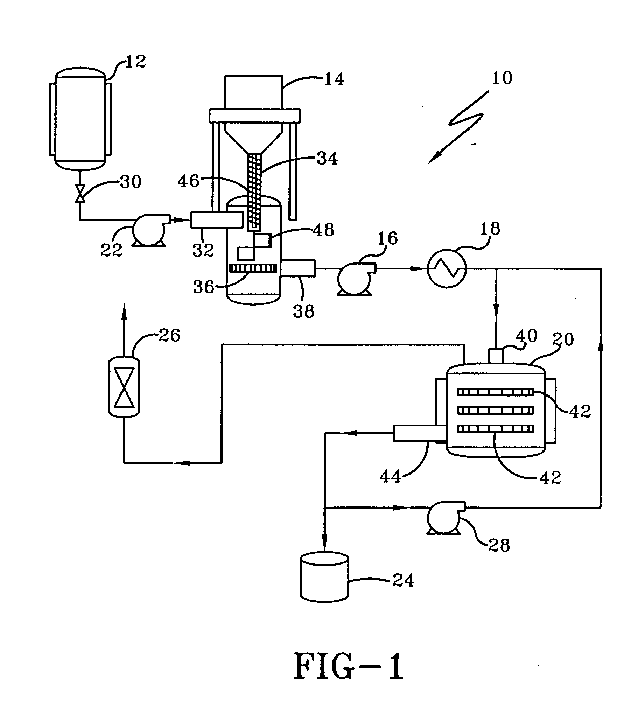

Referring now to the drawings wherein the showings are for purposes of illustrating the preferred embodiment of the invention only and not for purposes of limiting the same, *FIG. 1* shows a process **10** for making a metallic soap, in one embodiment being zinc stearate (preferably essentially clear) in which molten stearic acid is stored at ~170°F (77°C) in a heated first storage vessel **12.** When valve **30** is open, molten stearic acid is permitted to flow with the aid of pump **22** into slurry reactor inlet port **32** where it is mixed continuously with solid zinc oxide (ZnO) contained in storage vessel **14** for feeding via powder inlet tube **34** using a continuous liquid-solid mixer such as the MHD 2000 (Mixer ― Homogenizing ― Dispersing) commercially manufactured by IKA Works in Wilmington, North Carolina. This mixer provides continuous solid feeding which eliminates the aeration typically inherent in other suction type powder / liquid mixers. As better illustrated in *FIG*. 2, this mixer employs an auger **46** to feed the solids from solids inlet **34** with downstream liquids inlet **32** with mixing paddles **48** and rotor-stator combinations **36** prior to the slurry outlet **38** which is a homogeneous dispersed high solids concentration slurry.

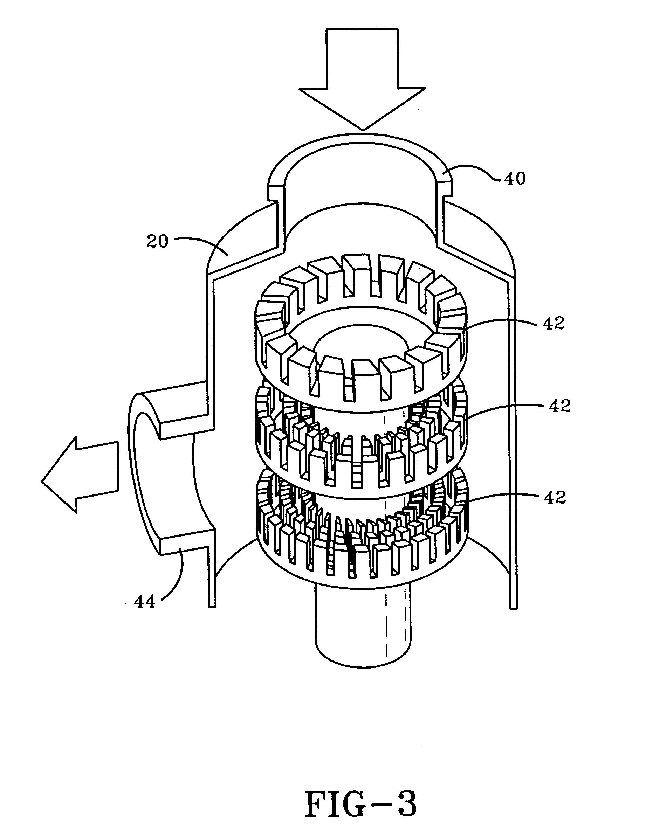

The continuous slurry stream is pumped using a centrifugal pump or positive displacement pump **16** and preheated to ~255°F (124°C), a temperature just below fusion initiation (~130°C) using a steam heater **18.** The preheated slurry feeds a continuous high shear reactor **20** where a micro-emulsion of the reactants is produced in a high energy, dispersing environment. A backpressure of 90 psig is maintained on reactor **20** to keep any liberated water from flashing. Under these conditions the reaction is essentially instantaneous and complete. Temperature rise across reactor **20** is ~30°F (17°C). As better illustrated in *FIG. 3,* high shear reactor **20** preferably employs at least one, preferably two or more rotator ― stator combinations **42** between reactor inlet **40** and product outlet **44.** In the most preferred embodiment, the rotor and stator combinations include three generators: one coarse combination for pre-crushing of coarse-grained suspensions; a medium combination often used as a universal mixing tool for emulsions and suspensions in the low to medium viscosity range; and a find combination which is especially suitable for micro-emulsions and suspensions which are reduced down to the smallest particle size.

The reaction mass continuously exiting the reactor is collected in receiver **24** operating at ~10 psig. Excess steam is vented in mist eliminator **26** leaving molten zinc stearate and optional recycle stream is pumped via pump **28** into reactor inlet **40.** Optionally, after filling the receiver, the 10 psig pressure is maintained for an additional ~30 minutes to eliminate any residual haze (if any) and produce a clear product. The remaining steam is then vented and the residual, clear product is suitable for finishing, e.g. prilling, flaking, etc. A nitrogen blanket is maintained over the molten product to maintain low color. Typical product is of outstanding quality: clear appearance, ash content 13.5%, free fatty acid 0.12%, water content 0.22% and melt point of 250°F (121°C). It should be recognized that in alternative embodiments more fully described below, residual haze is not present.

The advantages and important features of the present invention will be more apparent from the following examples.

### Example 1

Molten stearic acid at ~170°F (77°C) was pumped from storage through an in-line continuous mixer. Solid zinc oxide at ambient temperature was metered continuously from a super sack into the same continuous mixer at a rate so as to achieve approximate stoichiometric ratios of the two reactants. The slurry of zinc oxide in stearic acid exiting the mixer was then continuously preheated to ~255°F (124°C) in a shell-and-tube heat exchanger prior to entering the high shear, continuous reactor. A temperature rise of ~30°F (17°C) was noted in the high shear reactor. The continuous discharge from the reactor was routed through a standard pipe to a receiver. A backpressure of 90 psig was maintained on the pipe and reactor during the reaction. The reaction mass discharged continuously from the pipe into a receiver where byproduct steam was flashed to the vent system. Total residence time required for the reaction, i.e. from the high shear reactor through the pipe to the receiver, was ∼40 seconds. The combined feed rate of zinc oxide solids and stearic acid was maintained at 3,000 pounds per hour (1,361 Kg/hr) until 950 pounds (431 Kg) of ZnO and 6,515 pounds (2,892 Kg) of stearic acid were fed and the resulting zinc stearate product was collected in the receiver. The molten zinc stearate product had the following analysis: clear appearance, ash content 13.5%, free fatty acid 0.12%, water content 0.22%, and melt point of 250°F (121°C). The molten product was maintained under a nitrogen blanket to preserve color prior to being prilled (sprayed) to obtain the desired particle size, which in one embodiment, is about less than 100 microns. The characteristics of the zinc stearate were as shown in Table I.

**Table I**

| **Parameter** | **Prior Art** | **New process** |
|---|---|---|
| Yellowness Index | 13.2 | -0.1 |
| % Transmittance | 34.6 | 73.3 |
| Color (Gardner Index) | 4.1 G | < 1 G |

The Prior Art product was made in a batch mode by introducing stearic acid into a 1000 gallon stainless steel reactor having four longitudinally-directed spaced-apart baffles positioned about a periphery of the reactor which was further equipped with two vertically spaced-apart rotating impellers, each having three angled paddles, on a shaft rotating at about 88 revolutions per minute, tip speed of 481 ft/min (147 m/min), the reactor having an internal working pressure of 50 psig maximum at 350°F (177°C). After adding the requisite amount of stearic acid, an equimolar amount of solid zinc oxide is added into the reactor with agitation followed by heating the reactor to about 250°F (121°C). Allow the reaction to proceed for about 30 minutes followed by reducing the pressure followed by a nitrogen purge and collection of zinc stearate from the reactor.

### Example 2

A second run was conducted using the same operating conditions as example #1 in which 6,375 pounds (2,891 Kg) of stearic acid and 950 pounds (431 Kg) of zinc oxide were charged. The molten zinc stearate product had the following analysis: clear appearance, ash content 13.8%, free fatty acid 0.13%, water content 0.5%, and melt point of 250°F (121 °C).

### Example 3

A third run was conducted using the same operating conditions as example #1 in which 6,373 pounds (2,891 Kg) of stearic acid and 950 pounds (431 Kg) of zinc oxide were charged. The molten zinc stearate product had the following analysis: clear appearance, ash content 13.8%, free fatty acid 0.25%, water content 0.33%, and melt point of 250°F (121 °C).

As illustrated above, through the incorporation of a continuous slurry stream of stearic acid and zinc oxide, preheated to a temperature just below fusion initiation followed by entry into a continuous high shear reactor, a micro-emulsion of the reactants is produced in a high energy, dispersing environment to form a uniformly-sized metallic soap product. The residence time of this reaction is preferably under two (2) minutes, and more preferably under one (1) minute.

The metal salt reactants of this invention are the oxides, hydroxides, and carbonates of a wide variety of metals including sodium, potassium, lithium, magnesium, calcium, cadmium, strontium, barium, mercury, nickel, cobalt, lead, tin, nickel, iron, zinc, aluminum and copper. A single metal compound or a mixture of two or more of them can be used. However, when a clear salt is desired, zinc is preferred. Typical metallic compounds which may be used to produce the metallic fatty acid soap in accordance with this invention, a non-limiting exemplary list would include cadmium oxide, zinc carbonate, ferrous oxide, cadmium carbonate, calcium carbonate, calcium hydroxide, lead oxide, lead hydroxide, magnesium oxide, magnesium carbonate, cadmium hydroxide, zinc oxide, barium hydroxide, zinc hydroxide, etc.

The organic acid components useful in the practice of this invention include saturated and unsaturated aliphatic, aromatic, and alicyclic monocarboxylic, dicarboxylic, and polycarboxylic acids and the anhydrides of these acids. A non-limiting list of exemplary examples of the useful acids includes capric acid, lauric acid, myristic acid, palmitic acid, stearic acid, oleic, ricinoleic, arachidic acid, behenic acid, melissic acid, hydroxystearic acid, oxalic acid, succinic acid, glutaric acid, adipic acid, azelaic acid, sebacic acid, brassidic acid, erucic acid, petroselic acid, maleic acid, fumaric acid, sorbic acid, citraconic acid, mesaconic acid, itaconic acid, glutaconic acid, malic acid, tartaric acid, citric acid, aconitic acid, tricarballylic acid, tetrolic acid, benzoic acid, m-chlorobenzoic acid, p-chlorobenzoic acid, 2,4-dichlorobenzoic acid, 2,3,6-trichlorobenzoic acid, 2,3,6-tribromobenzoic acid, 2,3,5,6-tetrachlorobenzoic acid, 2,3,5,6-tetrabromobenzoic acid, p-aminobenzoic acid, 3,4-dimethoxybenzoic acid, p-tert-butylbenzoic acid, 2,6-dinitrobenzoic acid, salicyclic acid, p-hydroxybenzoic acid, 2,4-dihydroxybenzoic acid, gallic acid, phenylacetic acid, cinnamic acid, phthalic acid, isophthalic acid, terephthalic acid, trimellitic acid, trimesic acid, cyclohexanecarboxylic acid, cyclopentanecarboxylic acid, cyclopentane-1,2-dicarboxylic acid, abietic acid, and the like. Illustrative of the acid anhydrides that can be used are maleic anhydride, succinic anhydride, glutaric anhydride, cinnamic anhyride, benzoic anhydride, phthalic anhydride, 3-nitrophthalic anhydride, and tetrachlorophthalic anhydride as well as hydrogenated tallow fatty acids, the fatty acids of hydrogenated castor oil, coconut oil fatty acids and mixtures of the above with each other or with acids such as 9,10 oxylauric acid, 9,10 oxystearic acid, chlormethoxy stearic acid, 9,10 diketo stearic acid, phenyl stearic acid, etc., or palmitolic acid, stearolic acid behenolic acids, etc. Additionally, the fatty acid may be naturally derived from tallow, fish oil, sperm oil, coconut oil, palm oil, palm kernel oil, coco palm kernel oil, peanut oil, cottonseed oil, sunflower seed oil, soybean oil, linseed oil, rapeseed oil or stearine or synthetic fatty acids from paraffin oxidation. Such acids are well-known and are frequently used in the form of mixtures such as commercial stearic acid, lauric acid or the like. Commercial acids are mixtures of saturated and unsaturated fatty acids of varying carbon chains, typically ranging from C₄ - C₃₂, preferably from C₁₀ - C₂₂ and having a melting point above 0°C and below 150°C. Thus, what is illustrated is a carboxylic acid of formula (I) 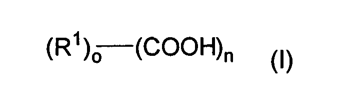 or an anhydride of formula (II) 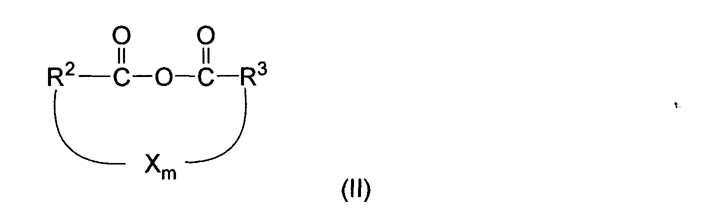 wherein R¹ (when present) is linear or branched, saturated or unsaturated, unsubstituted or substituted hydrocarbyl radicals of 2 - 32 carbon atoms. More specifically, R¹ is selected from the group consisting of C₂₋₃₂ alkyl, C₂₋₃₂ alkenyl, C₅₋₃₂ cycloalkyl, C₅₋₃₂ aryl, C₆₋₃₂ alkylaryl and C₆₋₃₂ arylalkyl, including branched and straight chain moieties and n is an integral value from 1 to 3 inclusive and substituted derivates thereof, said derivatives selected from the group consisting of hydroxyl, halogen, amine, C₂₋₆ alkyl amine, nitro and C₁₋₄ alkoxy and further wherein o is an integral value from 0 to 1 inclusive.

R² and R³ are linear or branched, saturated or unsaturated, unsubstituted or substituted hydrocarbyl radicals of 2 - 32 carbon atoms. More specifically, R² and R³ are independently selected from the group consisting of C₁₋₃₂ alkyl, C₂₋₃₂ alkenyl, C₅₋₃₂ cycloalkyl, C₅₋₃₂ aryl, C₆₋₃₂ alkylaryl and C₆₋₃₂ arylalkyl, including branched and straight chain moieties and substituted derivates thereof, said derivatives selected from the group consisting of hydroxyl, halogen, amine, C₂₋₆ alkyl amine, nitro and C₁₋₄ alkoxy and further wherein X is a covalent bond and m is an integral value from 0 to 1 inclusive.

The relative amounts of the metal component and the organic component that are in the reaction mixture are not critical. Equivalent amounts of the two components or a stoichiometric excess of the metal component is ordinarily used. In general, between 1% to 100% molar excess of the metal component in the reaction mixture is employed to insure that no undesired free acid groups remain after the reaction.

In a preferred embodiment, the metallic salt is added to the organic moiety which has been heated approximately to its melting temperature. The metallic salt powder is preferentially fed using a mixing - homogenizing - dispersing mixer capable of processing high solids content. In a more preferred embodiment, the design of the liquid solid mixer minimizes aeration and disperses high concentrations of difficult to wet out solids, even in viscous liquids.

While the above description has focused on the manufacture of a metallic ester comprised of essentially one metal, there is no need to limit the invention to such. In fact, it is envisioned to be within the scope of this invention to manufacture metallic esters containing more than one metal by the incorporation of more than one metal in the metal salt reactant. It is certainly within the scope of the invention to utilize two or more different metallic oxides or hydroxides or carbonates to form a mixed metal ester end product. It is also within the scope of the invention to utilize two or more different organic acid components.

The metallic salt / organic slurry is optionally preheated to a temperature which approximates (and typically is slightly lower than) the fusion initiation temperature of the reaction and fed into a high shear mixer. This high shear mixer is typically capable of forming micro-emulsions and very fine suspensions, typically due to the series of rotor-stator combinations which in series produce a small droplet or particle size with a narrow distribution. The nominal output revolutions per minute (rpm) typically range from 1,000 to 15,000 rpm with a nominal tip speed of from about 4,500 feet per minute (fpm) to 10,000 fpm. Tip speed, and therefore, shear rate, is an important factor in achieving the finest micro-emulsions.

Mixers that utilize a rotor and a stationary stator typically operate at considerably high rotational speeds that produce high rotor tip speeds. The differential speed between the rotor and the stator imparts extremely high shear and turbulent energy in the gap between the rotor and stator. Therefore, the tip speed is a very important factor when considering the amount of shear input into the product. Additionally, the gap distance between the rotor and the stator will contribute to the amount of shear. Another important factor is the shear frequency, or the number of occurrences that rotor and stator openings mesh.

In the foregoing description, certain terms have been used for brevity, clearness and understanding; but no unnecessary limitations are to be implied therefrom beyond the requirements of the prior art, because such terms are used for descriptive purposes and are intended to be broadly construed. Moreover, the description and illustration of the invention is by way of example, and the scope of the invention is not limited to the exact details shown or described.

This invention has been described in detail with reference to specific embodiments thereof, including the respective best modes for carrying out each embodiment. It shall be understood that these illustrations are by way of example and not by way of limitation.

## Claims

1. A process to make a metallic ester which comprises:
(a) mixing at least one metallic salt with at least one partially heated acidic organic moiety to form a heated slurry at a first temperature;
(b) reacting said slurry under shear conditions to form a micro-emulsion for no longer than about two minutes at a second higher temperature to form a pumpable metallic ester; and
(c) collecting said metallic ester.

2. The process of claim 1 wherein said step of reacting is preceded by heating said slurry to a higher temperature than said first temperature.

3. The process of either claim 1 or claim 2 wherein said at least partially heated acidic organic moiety is molten at said first temperature.

4. The process of any one of claims 1 to 3 wherein said step of reacting is no longer than about one minute.

5. The process of any one of claims 1 to 4 wherein said metallic salt and said acidic organic moiety are used in essentially stoichiometric amounts.

6. The process of any one of claims 1 to 4 wherein said metallic salt is in a stoichiometric excess to said acidic organic moiety from between 1% to 100% molar excess.

7. The process of any one of claims 1 to 6 wherein said shear conditions range from about 1,000 to 15,000 revolutions per minute.

8. The process of any one of claims 1 to 7 wherein at least one rotor and stator combination is used to generate said shear conditions.

9. The process of claim 8 wherein at least three rotor and stator combinations are used to produce a small droplet or particle size with a narrow distribution.

10. The process of any one of claims 1 to 9 wherein said metal of said metallic salt is selected from the group consisting of sodium, potassium, lithium, magnesium, calcium, cadmium, strontium, barium, mercury, nickel, cobalt, lead, tin, nickel, iron, zinc, aluminum and copper.

11. The process of claim 10 wherein said metal is zinc.

12. The process of any one of claims 1 to 11 wherein said salt is selected from the group consisting of oxides, hydroxides and carbonates.

13. The process of any one of claims 1 to 12 wherein said organic moiety is a carboxylic acid or an anhydride.

14. The process of claim 13 wherein
said carboxylic acid is of formula (I) 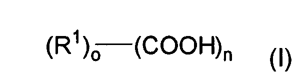 wherein
R¹ is a hydrocarbyl radical selected from the group consisting of linear, branched, saturated, unsaturated, unsubstituted and substituted hydrocarbyl radicals of 2 - 32 carbon atoms,
o is an integral value from 0 to 1 inclusive,
n is an integral value from 1 to 3 inclusive; and
said anhydride is of formula (11) 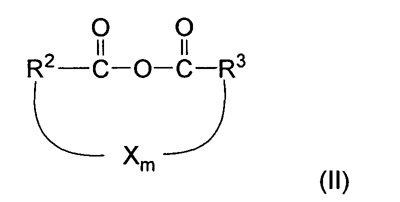 wherein
R² and R³ are independently hydrocarbyl radicals selected from the group consisting of linear, branched, saturated, unsaturated, unsubstituted and substituted hydrocarbyl radicals of 2 - 32 carbon atoms,
X is a covalent bond; and
m is an integral value from 0 to 1 inclusive.

15. The process of claim 14 wherein
R¹ is selected from the group consisting of C₂₋₃₂ alkyl, C₂₋₃₂ alkenyl, C₅₋₃₂ cycloalkyl, C₅₋₃₂ aryl, C₆₋₃₂ alkylaryl and C₆₋₃₂ arylalkyl and substituted derivates thereof, wherein said derivatives are selected from the group consisting of hydroxyl, halogen, amine, C₂₋₆ alkyl amine, nitro and C₁₋₄ alkoxy; and
R² and R³ are independently selected from the group consisting of C₁₋₃₂ alkyl, C₂₋₃₂ alkenyl, C₅₋₃₂ cycloalkyl, C₅₋₃₂ aryl, C₆₋₃₂ alkylaryl and C₆₋₃₂ arylalkyl and substituted derivates thereof, said derivatives selected from the group consisting of hydroxyl, halogen, amine, C₂₋₆ alkyl amine, nitro and C₁₋₄ alkoxy.

16. The process of any one of claims 1 to 9 wherein said metallic salt is zinc oxide and said organic moiety is stearic acid.

17. The process of claim 16 in which the reaction conditions are such that there is prepared zinc stearate having a Gardner Index of less than 1.

18. The process of claim 17, which comprises:
(a) mixing a zinc salt with at least partially molten stearic acid in essentially stoichiometric amounts to form a heated slurry at a first temperature;
(b) reacting said slurry under shear conditions to form a micro-emulsion for less than about two minutes at a second higher temperature to form a pumpable zinc stearate; and
(c) collecting said zinc stearate.
